# DEMANDE DE BREVET EUROPEEN

(11) **EP 1 721 896 A1**
(43) Date de publication de la demande: **15.11.2006**
(21) Numéro de dépôt: 06290757.1
(22) Date de dépôt: 11.05.2006
(51) Int. Cl.: C07D 213/72, A61K 31/44

(54) **Dérivés de phénylpyridinylpiperazine, leur procédé de préparation et les compositions pharmaceutiques qui les contiennent**

(30) Priorité: 12.05.2005 FR 0504757
(71) Demandeur: Les Laboratoires Servier, 92415 Courbevoie Cedex (FR)
(72) Inventeur: Desos, Patrice, 92270 Bois-Colombes (FR); Cordi, Alexis, 92150 Suresnes (FR); Lestage, Pierre, 78170 La Celle-St-Cloud (FR)

(57) **Abrégé**

Composés de formule (I) : 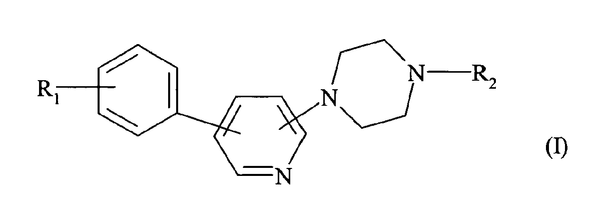 dans laquelle :
R₁ représente un groupement NR₃SO₂R₄ dans lequel :
R₃ représente un atome d'hydrogène ou un groupement alkyle
R₄ représente un groupement alkyle, un groupement aryle ou un groupement NR₅R₆
R₂ représente un groupement alkyle, cycloalkyle ou cycloalkylalkyle,

Médicaments.

## Description

La présente invention concerne de nouveaux dérivés de phénylpyridinylpipérazine, leur procédé de préparation et les compositions pharmaceutiques qui les contiennent.

Les composés de la présente invention sont particulièrement intéressants d'un point de vue pharmacologique pour leur interaction spécifique avec les récepteurs histaminergiques centraux de type H₃, trouvant leur application dans le traitement des neuropathologies associées au vieillissement cérébral, des troubles de l'humeur, du comportement alimentaire et du rythme veille-sommeil, ainsi que du syndrome d'hyperactivité avec déficits attentionnels.

Le vieillissement de la population par augmentation de l'espérance de vie à la naissance a entraîné parallèlement un large accroissement de l'incidence des neuropathologies liées à l'âge et notamment de la maladie d'Alzheimer. Les principales manifestations cliniques du vieillissement cérébral et surtout des neuropathologies liées à l'âge, sont les déficits des fonctions mnésiques et cognitives qui peuvent conduire à la démence.

Au niveau du système nerveux central, de récentes études neuropharmacologiques ont montré que l'histamine *via* les systèmes histaminergiques centraux jouait un rôle de neurotransmetteur ou neuromodulateur en situations physiologiques ou physiopathologiques (Annu. Rev. Neurosci., 1986, 9, 209-254; Physiol. Rev., 1991, 71, 1-51). Ainsi, il a été montré que l'histamine intervenait dans divers processus physiologiques et comportementaux tels que la thermorégulaton, la régulation neuro-endocrinienne, le rythme circadien, les états cataleptiques, la motricité, l'agressivité, le comportement alimentaire, l'apprentissage et la mémorisation, ainsi que la plasticité synaptique (Hass et coll., histaminergic neurones : morphology and function, Boca Raton, FL: CRC Press, 1991, pp. 196-208; Prog. Neurobiology, 2001, 63, 637-672).

Parmi les 3 sous-types de récepteurs (H₁, H₂ et H₃) à l'histamine, il a été initialement montré que le récepteur de type H₃ était un autorécepteur pré-synaptique qui contrôlait la libération de l'histamine (Nature, 1987, 327, 117-123). Son activation inhibe la libération et la synthèse d'histamine par un mécanisme de feedback négatif (Neuroscience, 1987, 23, 149-157). Par la suite, il a été montré l'existence d'hétérorécepteurs pré-synaptiques, capables de moduler la libération de quelques neuropeptides et de nombreux neurotransmetteurs tels que noradrénaline, sérotonine, dopamine, GABA, acétylcholine et glutamate (TiPS, 1998, 19, 177-183). Des études, réalisées chez l'animal, ont montré que l'augmentation des taux endogènes extra-synaptiques d'histamine *via* le blocage des récepteurs de type H₃ par des antagonistes H₃ permettait de promouvoir les états de vigilance, les processus d'apprentissage et de mémoire, de réguler la prise alimentaire, et de s'opposer à des crises convulsives (Prog. Neurobiol., 2000, 63, 637-672; Neurosci. Biobehav. Rev., 2000, 24, 107-113). En conséquence, les indications thérapeutiques potentielles pour des antagonistes H₃ sont le traitement des déficits cognitifs associés au vieillissement cérébral et aux maladies neurodégénératives telles que la maladie d'Alzheimer, la maladie de Parkinson, la maladie de Pick, la maladie de Korsakoff et les démences frontales ou sous-corticales d'origine vasculaires ou autres, ainsi que le traitement des troubles de l'humeur, des crises convulsives, du syndrome d'hyperactivité avec déficits attentionnels, de l'obésité, de la douleur et des états narcoleptiques.

Les composés de la présente invention, outre leur originalité structurale, présentent des propriétés pharmacologiques tout à fait surprenantes et intéressantes dans ce domaine.

Plus spécifiquement, la présente invention concerne les composés de formule (I) : 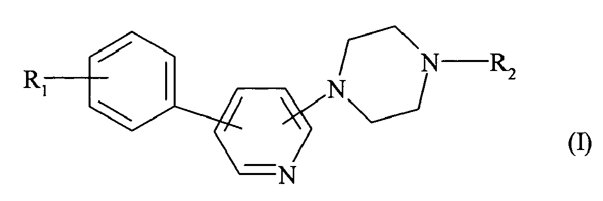 dans laquelle :
- R₁: représente un groupement NR₃SO₂R₄ dans lequel :
- R₃ représente un atome d'hydrogène ou un groupement alkyle (C₁-C₆) linéaire ou ramifié,
- R₄ représente un groupement alkyle (C₁-C₆) linéaire ou ramifié, un groupement aryle ou un groupement NR₅R₆ dans lequel :
   R₅ et R₆, identiques ou différents, représentent chacun un atome d'hydrogène ou un groupement alkyle (C₁-C₆) linéaire ou ramifié, cycloalkyle (C₃-C₈) ou cycloalkyl (C₃-C₈) alkyle (C₁-C₆) linéaire ou ramifié,
   ou R₅ et R₆ forment ensemble avec l'atome d'azote qui les porte un cycle comportant de 5 à 8 chaînons dont un des atomes de carbone peut être remplacé par un atome d'azote, d'oxygène ou de soufre, ou par un groupement SO ou SO₂,
le cycle ainsi défini pouvant être ponté par une chaîne alkyle(C₁-C₆) linéaire ou ramifiée et/ou pouvant être éventuellement substitué par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, carboxy, hydroxy, cyano, nitro, amino (éventuellement substitué par un ou plusieurs groupements alkyle (C₁-C₆) linéaire ou ramifié),
- R₂: représente un groupement alkyle (C₁-C₆) linéaire ou ramifié, un groupement cycloalkyle (C₃-C₈), ou un groupement cycloalkyle (C₃-C₈) alkyle (C₁-C₆) dans lequel la partie alkyle peut être linéaire ou ramifiée,
étant entendu que :
par groupement aryle, on comprend les groupements phényle, naphtyle et biphényle, ces groupements étant éventuellement substitués par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, carboxy, hydroxy, cyano, nitro, amino (éventuellement substitué par un ou plusieurs groupements alkyle (C₁-C₆) linéaire ou ramifié),
leurs énantiomères et diastéréoisomères ainsi que leurs sels d'addition à un acide ou à une base pharmaceutiquement acceptable.

Parmi les acides pharmaceutiquement acceptables, on peut citer, à titre non limitatif, les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthane sulfonique, camphorique, etc...

Parmi les bases pharmaceutiquement acceptables, on peut citer, à titre non limitatif, l'hydroxyde de sodium, l'hydroxyde de potassium, la triéthylamine, la tertbutylamine, etc...

Plus particulièrement, l'invention concerne les composés de formule (I) pour lesquels R₄ représente un groupement alkyle, comme le groupement méthyle par exemple.

Le groupement R₃ préféré est l'atome d'hydrogène.

Avantageusement, l'invention concerne les composés de formule (I) pour lesquels R₅ et R₆ forment ensemble avec l'atome d'azote qui les porte un cycle comportant de 5 à 8 chaînons dont un des atomes de carbone peut être remplacé par un atome d'azote, d'oxygène ou de soufre ou par un groupement SO ou SO₂ comme le groupement morpholine par exemple.

Les groupements R₂ préférés sont les groupements isopropyle, cyclopropyle et cyclopentyle.

Encore plus particulièrement, l'invention concerne les composés de formule (I) qui sont :
- le *N*-{4-[6-(4-isopropylpipérazin-1-yl)pyridin-3-yl]phényl}benzènesulfonamide dichlorhydrate,
- le *N*-(4-[6-(4-cyclopentylpipérazin-1-yl)pyridin-3-yl]phényl)benzènesulfonamide dichlorhydrate,
- le *N*-[4-[6-(4-cyclopentyl-1-pipérazinyl)-3-pyridinyl]phényl]-méthanesulfonamide, dichlorhydrate,
- le *N*-{4-[6-(4-cyclopropylpipérazin-1-yl)pyridin-3-yl]phényl}méthanesulfonamide dihydrochloride,
- le *N*-{4-[6-(4-isopropylpipérazin-1-yl)pyridin-3-yl]phényl}morpholme-4-sulfonamide dichlorhydrate,
- N-{4-[6-(4-isopropylpipérazin-1-yl)pyridin-3-yl]phényl}méthanesulfonamide dichlorhydrate,
- *N*-{4-[6-(4-isopropylpipérazin-1-yl)pyridin-3-yl]phényl}propane-2-sulfonamide dichlorhydrate,
- *N*-{4-[6-(4-cyclopentylpipérazin-1-yl)pyridin-3-yl]phényl}-4-fluorobenzène sulfonamide dichlorhydrate,
- *N*-{4-[6-(4-cyclopentylpipérazin-1-yl)pyridin-3-yl]phényl}-3-fluorobenzène sulfonamide dichlorhydrate,
- *N*-{4-[6-(4-cyclopentylpipérazin-1-yl)pyridin-3-yl]phényl}-2-fluorobenzène sulfonamide dichlorhydrate.

L'invention concerne également le procédé de préparation des composés de formule (I) caractérisé en ce que l'on utilise comme produit de départ le composé de formule (II) : 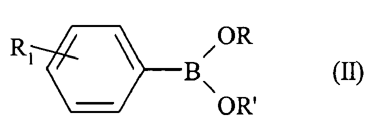 dans laquelle R₁ est tel que défini dans la formule (I) et R et R', identiques ou différents, représentent chacun un atome d'hydrogène ou un groupement alkyle (C₁-C₆) linéaire ou ramifié, ou forment ensemble une chaîne alkylène (C₁-C₆) linéaire ou ramifiée,
sur lequel on condense en présence de palladium (0) un composé de formule (III) : 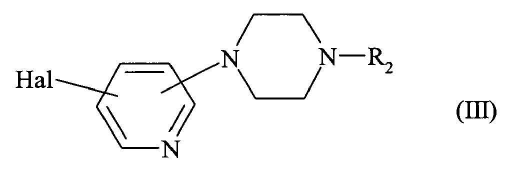 dans laquelle R₂ est tel que défini dans la formule (I) et Hal représente un atome d'halogène, pour conduire au composé de formule (I),
composé de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

Les composés de formule (II) et (III), tels que définis précédemment, sont commerciaux ou obtenus par des réactions classiques de la chimie organique.

Les composés de la présente invention, de par leurs propriétés pharmacologiques de ligands histaminiques du récepteur H₃, sont utiles dans le traitement des déficits cognitifs associés au vieillissement cérébral et aux maladies neurodégénératives telles que la maladie d'Alzheimer, la maladie de Parkinson, la maladie de Pick, la maladie de Korsakoff et les démences frontales ou sous-corticales d'origine vasculaires ou autres, ainsi que le traitement des troubles de l'humeur, des crises convulsives, du syndrome d'hyperactivité avec déficits attentionnels, de l'obésité, de la douleur et des états narcoleptiques.

La présente invention a également pour objet les compositions pharmaceutiques renfermant comme principe actif au moins un composé de formule (I), un de ses isomères, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 10 mg à 1 g en une ou plusieurs prises par jour.

Les préparations et exemples suivants illustrent l'invention mais ne la limitent en aucune façon.
Les produits de départ utilisés sont des produits connus ou préparés selon des modes opératoires connus.
Les structures des composés, décrits dans les exemple, ont été déterminées selon les techniques spectrophotométriques usuelles (infrarouge, RMN, spectrométrie de masse...).

### Préparation 1 : 1-(5-Bromopyridin-2-yl)-4-isopropylpipérazine

Une solution contenant 12,1 g de 2,5-dibromopyridine (51,1 mmol), 8,8 ml de 1-isopropylpipérazine (61,5 mmol) et 9,2 ml de DBU (61,5 mmol) est agitée une nuit à 100 °C. La réaction est ramenée à température ambiante et la solution diluée dans de l'eau et extraite par de l'acétate d'éthyle. Les phases organiques sont rassemblées, lavées par de la saumure, séchées (MgSO₄) et évaporées sous pression réduite. Le résidu est chromatographié sur colonne de SiO₂ en éluant par un mélange CH₂Cl₂/MeOH 98/2 puis 96/4 pour conduire au produit du titre.
*Point de fusion : 76-78 °C*
*Microanalyse élémentaire :*

| | *C* | *H* | *N* | *Br* |
|---|---|---|---|---|
| *% théorique* | *50,72* | *6,38* | *14,79* | *28,12* |
| *% expérimental* | *50,.96* | *6,.47* | *14,53* | *28,33* |

### Préparation 2 : 1-(5-Bromopyridin-2-yl)-4-cyclopentylpipérazine

Mode opératoire identique à celui de la Préparation 1 en remplaçant la 1-isopropylpipérazine par la 1-cyclopentylpiperazine.
*Point de fusion : 127-128 °C*

### Préparation 3 : 1-(5-Bromopyridin-2-yl)-4-cyclopropylpipérazine

Mode opératoire identique à celui de la Préparation 1 en remplaçant la 1-isopropylpipérazine par la 1-cyclopropylpiperazine.
*Point de fusion : 110-115 °C*

### Exemple 1 : N-{4-[6-(4-Isopropylpipérazin-1-yl)pyridin-3-yl]-phényl}benzènesulfonamide dichlorhydrate

### Stade A : N-(4-Iodophényl)benzènesulfonamide

A une solution de 2,0 g de 4-iodoaniline (9,13 mmol) dans 40 ml d'acétonitrile sont rajoutés 1,48 ml de pyridine (18,26 mmol) puis, goutte à goutte, une solution de 1,28 ml de chlorure de benzènesulfonyle (10 mmol) dans 20 ml d'acétonitrile. La réaction est agitée une nuit à température ambiante et l'acétonitrile est évaporé sous pression réduite. Le résidu est repris dans HCl 1N et extrait par l'acétate d'éthyle. La phase organique est lavée par de la saumure, séchée (MgSO₄), évaporée sous pression réduite. Le résidu huileux obtenu est trituré dans de l'éther isopropylique jusqu'à cristallisation du produit du titre.
*Point de fusion : 141-143 °C*

### Stade B : N-[4-(4,4,5,5-Tétraméthyl-1,3,2-dioxaborolan-2-yl)phényl]benzène sulfonamide

Dans un bicol de 25 ml sont introduits 500 mg du composé obtenu au Stade A (1,39 mmol), 389 mg de bis-pinacolatodiborane (1,53 mmol), 410 mg d'acétate de potassium (4,18 mmol) et 5 ml de diméthylformamide. Le milieu réactionnel est dégazé en barbotant un courant d'azote pendant 30 min, puis 16 mg d'acétate de palladium (0,07 mmol) sont ajoutés. La réaction est agitée sous léger courant d'azote pendant 7 heures à 85 °C. Après refroidissement à température ambiante, le milieu réactionnel est dilué dans de l'eau et extrait par de l'acétate d'éthyle. Les phases organiques sont réunies, lavées par de la saumure, séchées, évaporées sous pression réduite. Le résidu d'évaporation est trituré dans l'heptane pour conduire, après filtration, au produit du titre sous la forme d'un solide blanc.
*Point de fusion : 157-160* °C

### Stade C : N-{4-[6-(4-Isopropylpipérazin-1-yl)pyridin-3-yl]phényl}benzène sulfonamide dichlorhydrate

Dans un bicol de 25 ml sont introduits 226mg du composé obtenu dans la Préparation 1 (0,79 mmol), 300 mg du composé obtenu au Stade B (0,83 mmol), 3 ml de dioxanne et 3 ml de Na₂CO₃ 0,4 M aqueux. Le milieu réactionnel est dégazé en faisant buller de l'azote pendant 30 min. Le Pd(0)tétrakistriphénylphosphine (45 mg, 0,04 mmol) est introduit et la réaction est agitée à 90 °C sous léger courant d'azote pendant 3 heures. Après refroidissement à température ambiante, le milieu réactionnel est dilué dans l'eau et extrait par de l'acétate d'éthyle. Les phases d'extractions sont jointes, lavées par de la saumure, séchée (MgSO₄) et évaporées sous pression réduite. Le résidu obtenu est chromatographié sur SiO₂ (CH₂Cl₂/MeOH/NH₄OH 96/4/0,4) pour conduire au produit du titre sous forme de base. Celle ci est dissoute dans de l'éther chlorhydrique, puis la solution est concentrée et filtrée pour conduire au produit du titre sous forme de chlorhydrate.
*Point de fusion : 160-163 °C*
*Microanalyse élémentaire :*

| | *C* | *H* | *N* | *S* | *Cl* |
|---|---|---|---|---|---|
| *% théorique* | *56,58* | *5,93* | *11,00* | *6,29* | *13,92* |
| *% expérimental* | *55,36* | *6,3* | *10, 62* | *6,32* | *13, 67* |

### Exemple 2 : N-{4-[6-(4-Cyclopentylpipérazin-1-yl)pyridin-3-yl]phényl}benzène sulfonamide dichlorhydrate

Le produit obtenu dans le Stade B de l'Exemple 1 est mis en réaction avec le composé obtenu dans la Préparation 2 selon les conditions décrites au Stade C de l'Exemple 1.
*Point de fusion : 162-167 °C C*
*Microanalyse élémentaire :*

| | *C* | *H* | *N* | *S* | *Cl* |
|---|---|---|---|---|---|
| *% théorique* | *58,31* | *6, 02* | *10,46* | *5,99* | *13, 24* |
| *% expérimental* | *58,71* | *6,05* | *10,53* | *6, 03* | *12, 66* |

### Exemple 3 : N-[4-[6-(4-Cyclopentyl-1-pipérazinyl)-3-pyridinyl]phényl]-méthane sulfonamide, dichlorhydrate

### Stade A : N-(4-Iodophényl)méthanesulfonamide

Mode opératoire identique au stade A de l'Exemple 1 mais en remplaçant le chlorure de benzènesulfonyle par l'anhydride méthanesulfonique.
*Point de fusion : 118-120 °C*
*Microanalyse élémentaire :*

| | *C* | *H* | *N* | *S* | *I* |
|---|---|---|---|---|---|
| *% théorique* | *28,30* | *2,71* | *4,71* | *10,79* | *42,71* |
| *% expérimental* | *28, 67* | *2,83* | *4,70* | *11, 22* | *43,44* |

### Stade B : N-[4-(4,4,5,5-Tétraméthyl-1,3,2-dioxaborolan-2-yl)phényl]méthane sulfonamide

Mode opératoire identique au stade B de l'Exemple 1 mais à partir du produit obtenu dans le Stade A précédent.
*Point de fusion : 180-182 °C*

### Stade C : N-[4-[6-(4-Cyclopentyl-1-pipérazinyl)-3-pyridinyl]phényl]méthane sulfonamide, dichlorhydrate

Mode opératoire identique au Stade C de l'Exemple 1 à partir du produit obtenu au Stade B et en remplaçant le composé obtenu dans la Préparation 1 par le composé obtenu dans la Préparation 2.
*Point de fusion : 227-229 °C*
*Microanalyse élémentaire :*

| | *C* | *H* | *N* | *S* | *Cl* |
|---|---|---|---|---|---|
| *% théorique* | *53,27* | *6,39* | *11,83* | *6,77* | *14,98* |
| *% expérimental* | *53,22* | *6,41* | *11,43* | *6,85* | *14,89* |

### Exemple 4 : N-{4-[6-(4-Cyclopropylpipérazin-1-yl)pyridin-3-yl]phényl}méthane sulfonamide dichlorhydrate

Le produit obtenu dans le Stade B de l'Exemple 3 est mis en réaction avec le composé obtenu dans la Préparation 3 selon les conditions décrites au Stade C de l'Exemple 1.
*Point de fusion : 197°C*
*Microanalyse élémentaire :*

| | *C* | *H* | *N* | *S* | *Cl* |
|---|---|---|---|---|---|
| *% théorique* | *51, 24* | *5,88* | *12,58* | *7, 20* | *15,92* |
| *% expérimental* | *51,70* | *5,78* | *12,21* | *6,81* | *15,93* |

### Exemple 5 : N-{4-[6-(4-Isopropylpipérazin-1-yl)pyridin-3-yl]phényl}morpholine-4-sulfonamide dichlorhydrate

### Stade A : N-(4-Iodophényl)morpholine-4-sulfonamide

A une solution de 10 g de 4-iodoaniline (45,6 mmol) dans 200 ml d'acétonitrile sont rajoutés 6,41 ml de Et₃N (45,6 mmol) et 8,47g de chlorure de morpholine-4-sulfonyle (45,6 mmol). La réaction est agitée 16 heures à température ambiante. L'acétonitrile est évaporé sous vide et le résidu repris dans HCl 1N et extrait par CH₂Cl₂. Les phases organiques sont réunies, lavées par de la saumure, séchée (MgSO₄) et traitées au noir animal pour conduire au produit du titre.
*Point de fusion : 91 °C*

### Stade B : N-[4-(4,4,5,5-Tétraméthyl-1,3,2-dioxaborolan-2-yl)phényl] morpholine-4-sulfonamide

Mode opératoire identique au Stade B de l'Exemple 1 à partir du produit obtenu dans le Stade A.
*Point de fusion : 158-161 °C*

### Stade C : N-{4-[6-(4-Isopropylpipérazin-1-yl)pyridin-3-yl]phényl}morpholine -4-sulfonamide dichlorhydrate

Mode opératoire identique au Stade C de l'Exemple 1 à partir du produit obtenu dans le Stade B.
*Point de fusion : 194-198 °C*
*Microanalyse élémentaire :*

| | *C* | *H* | *N* | *S* | *Cl* |
|---|---|---|---|---|---|
| *% théorique* | *50,96* | *6,41* | *13,51* | *6,18* | *13, 67* |
| *% expérimental* | *50,90* | *6,79* | *13,23* | *6,09* | *13,46* |

### Exemple 6 : N-{4-[6-(4-Isopropylpipérazin-1-yl)pyridin-3-yl]phényl}méthane sulfonamide dichlorhydrate

Le produit du stade B de l'Exemple 3 est mis en réaction avec le composé obtenu dans la Préparation 1 selon les conditions décrites au Stade C de l'Exemple 1.
*Point de fusion : 191 °C*
*Microanalyse élémentaire :*

| | *C* | *H* | *N* | *S* | *Cl* |
|---|---|---|---|---|---|
| *% théorique* | *50,19* | *6, 25* | *12,32* | *7,05* | *17,15* |
| *% expérimental* | *50,14* | *6,10* | *11,49* | *6,58* | *17,25* |

### Exemple 7 : N-{4-[6-(4-Isopropylpipérazin-1-yl)pyridin-3-yl]phényl}propane-2-sulfonamide dichlorhydrate

### Stade A : N-(4-Iodophényl)propane-2-sulfonamide

Mode opératoire identique au Stade A de l'Exemple 5 en remplaçant le chlorure de morpholine-4-sulfonyle par le chlorure de propane-2-sulfonyle.
*Point de fusion : 96 °C*
*Microanalyse élémentaire :*

| | *C* | *H* | *N* | *S* | *I* |
|---|---|---|---|---|---|
| *% théorique* | *33, 24* | *3,72* | *4,31* | *9,86* | *39, 03* |
| *% expérimental* | *33,21* | *3,38* | *4,17* | *9,74* | *38,37* |

### Stade B : N-[4-(4,4,5,5-Tétraméthyl-1,3,2-dioxaborolan-2-yl)phényl]propane-2-sulfonamide

Mode opératoire identique au Stade B de l'Exemple 1, à partir du produit obtenu au Stade A.
*Point de fusion : 192 °C*
*Microanalyse élémentaire :*

| | *C* | *H* | *N* | *S* |
|---|---|---|---|---|
| *% théorique* | *55,40* | *7,44* | *4,31* | *9,86* |
| *% expérimental* | *55,46* | *7,33* | *4,54* | *10,11* |

### Stade C : N-{4-[6-(4-Isopropylpipérazin-1-yl)pyridin-3-yl]phényl}propane-2-sulfonamide dichlorhydrate

Mode opératoire identique au Stade C de l'Exemple 1, à partir du produit obtenu au Stade B.
*Point de fusion : 165 °C*
*Microanalyse élémentaire :*

| | *C* | *H* | *N* | *S* | *Cl* |
|---|---|---|---|---|---|
| *% théorique* | *53,05* | *6,78* | *11,78* | *6,74* | *14,91* |
| *% expérimental* | *53,05* | *7,07* | *11,58* | *6,47* | *14,62* |

### Exemple 8 : N-{4-[6-(4-Cyclopentylpipérazin-1-yl)pyridin-3-yl]phényl}-4-fluorobenzènesulfonamide dichlorhydrate

### Stade A : {4-[6-(4-Cyclopentylpipérazin-1-yl)pyridin-3-yl]phényl}amine

Mode opératoire identique au Stade C de l'Exemple 1 en utilisant la [4-(4,4,5,5-tétraméthyl-1,3,2-dioxaborolan-2-yl)phényl]amine.
*Point de fusion : 160-162 °C*

### Stade B : N-{4-[6-(4-Cyclopentylpipérazin-1-yl)pyridin-3-yl]phényl}-4-fluorobenzènesulfonamide dichlorhydrate

Une suspension contenant 200 mg (0,620 mmol) du produit obtenu au Stade A et 302 mg (1,55 mmol) du chlorure de l'acide 4-fluorophényl sulfonique dans 2 ml de pyridine est agitée 3h à 80°C. Après refroidissement à température ambiante, le milieu réactionnel est précipité par addition d'eau. Le précipité est récupéré par filtration, dissous dans un mélange CH₂Cl₂/MeOH, absorbé sur environ 1 g de silice et chromatographié sur colonne de silice en éluant avec un mélange CH₂Cl₂/MeOH/NH₃ 98/2/0,2. Le dichlorhydrate est formé par reprise de la base dans l'éthanol et addition d'éther chlorhydrique.
*Point de fusion : 256-262 °C*
*Microanalyse élémentaire :*

| | *C* | *H* | *N* | *S* | *Cl* |
|---|---|---|---|---|---|
| *% théorique* | *56,42* | *5, 64* | *10,12* | *5,79* | *12,81* |
| *% expérimental* | *56, 03* | *5,73* | *9,77* | *5,45* | *12,74* |

### Exemple 9 : N-{4-[6-(4-Cyclopentylpipérazin-1-yl)pyridin-3-yl]phényl}-3-fluorobenzènesulfonamide dichlorhydrate

Mode opératoire identique à l'Exemple 8 mais en utilisant au Stade B le chlorure de l'acide 3-fluorophényl sulfonique.
*Point de fusion : 167-170 °C*
*Microanalyse élémentaire :*

| | *C* | *H* | *N* | *S* | *Cl* |
|---|---|---|---|---|---|
| *% théorique* | *56,42* | *5,64* | *10,12* | *5, 79* | *12,81* |
| *% expérimental* | *56,72* | *5,60* | *9,96* | *5, 67* | *12,86* |

### Exemple 10 : N-{4-[6-(4-Cyclopentylpipérazin-1-yl)pyridin-3-yl]phényl}-2-fluorobenzènesulfonamide dichlorhydrate

Mode opératoire identique à l'Exemple 8 mais en utilisant au Stade B le chlorure de l'acide 2-fluorophényl sulfonique.
*Point de fusion : 248-253 °C*
*Microanalyse élémentaire :*

| | *C* | *H* | *N* | *S* | *Cl* |
|---|---|---|---|---|---|
| *% théorique* | *56,42* | *5,64* | *10,12* | *5,79* | *12,81* |
| *% expérimental* | *56,46* | *5,63* | *9,87* | *5,47* | *12,80* |

### ETUDE PHARMACOLOGIQUE DES PRODUITS DE L'INVENTION

### EXEMPLE A : Dosages cérébraux de la N^{τ}-Méthylhistamine chez la souris NMRI

Cette étude réalisée selon la méthode de Taylor et Coll. (Biochem. Pharm., 1992, 44, 1261-1267), a pour objectif d'évaluer l'activité *ex vivo* des composés de la présente invention en tant qu'antagonistes des récepteurs histaminergiques centraux de type H₃. Cette activité est révélée par la mesure, après traitement par voie intrapéritonéale des composés sous étude, des taux centraux de N^{τ}- Méthylhistamine, métabolite principal de l'histamine. Une augmentation des concentrations cérébrales de N^{τ}- Méthylhistamine signe une augmentation du turn-over de l'histamine par blocage des récepteurs histaminergiques centraux de type H₃.

Des souris NMRI (18-20g) sont traitées par voie intrapéritonéale ou orale par les composés de la présente invention ou par leur véhicule (20 ml/kg). Une heure après le traitement pharmacologique, les animaux sont sacrifiés, les cerveaux sont prélevés, congelés dans l'azote liquide, pesés et homogénéisés dans HClO₄ 0,1N à 4°C. Les homogénats sont centrifugés (15000g, 17 min, 4°C). Les surnageants sont récupérés et aliquotés. Les aliquotes sont congelés dans l'azote liquide et stockés à -80°C jusqu'à leur analyse. La détermination des taux cérébraux de N^{τ}- Méthylhistamine est réalisée par dosage radio-immunologique (RIA) à l'aide d'un kit de dosage. Les taux tissulaires de N^{τ}-Méthylhistamine sont exprimés en µg/g de cerveau frais. La comparaison des taux cérébraux de N^{τ-}Méthylhistamine entre les animaux traités par le véhicule (témoins) et les animaux traités par les composés de la présente invention est effectuée par une analyse de variance à un facteur suivie si nécessaire par une analyse complémentaire (test de Dunnett).

Les résultats montrent que les composés de la présente invention sont capables, pour des doses comprises entre 1 et 10 mg/kg PO, d'augmenter de 100% les concentrations cérébrales endogènes de N^{τ}-Méthylhistamine.
A titre d'exemple, les composés des Exemples 4 et 7 administrés respectivement à 10 et 3 mg/kg PO permettent d'obtenir une augmentation de 162 % et 138 % respectivement des concentrations cérébrales endogènes de N^{τ}-Méthylhistamine.

Ces résultats indiquent que les composés de la présente invention sont de puissants antagonistes des récepteurs histaminergiques centraux de type H₃.

### EXEMPLE B : Enregistrements de l'électroencephalogramme chez le rat vigile

Des rats Wistar mâles adultes sont implantés de manière chronique par des électrodes placées à la surface du cortex frontal et pariétal. Des enregistrements de l'électroencephalogramme (EEG) cortical sont effectués chez les rats placés dans des cages dans une pièce isolée phoniquement. Les composés et solvant sont administrés de manière randomisée à 10 h les mêmes jours avec un minimum de 3 jours entre chaque administration, permettant d'utiliser chaque rat comme son propre témoin. La puissance absolue des activités lentes delta (1-4 Hz), qui prédominent pendant le sommeil lent et disparaissent pendant l'éveil et le sommeil paradoxal, est moyennée sur des périodes successives de 30 min. Sur 30 min, des valeurs élevées et basses de la puissance des activités lentes delta sont des signes d'éveil et de sommeil, respectivement.
Les résultats montrent que les composés de la présente invention augmentent l'éveil (diminution des ondes delta) pour des doses comprises entre 0,3 et 3 mg/kg IP.

### EXEMPLE C : Composition pharmaceutique

| | |
|---|---|
| Formule de préparation pour 1000 comprimés dosés à 100 mg de *N*-[4-[6-(4-cyclopentyl-1-pipérazinyl)-3-pyridinyl]phényl]méthane sulfonamide, dichlorhydrate (exemple 3) | 100 g |
| Hydroxypropycellulose | 2 g |
| Amidon de blé | 10 g |
| Lactose | 100 g |
| Stéarate de magnésium | 3 g |
| Talc | 3 g |

## Revendications

1. Composés de formule (I) : 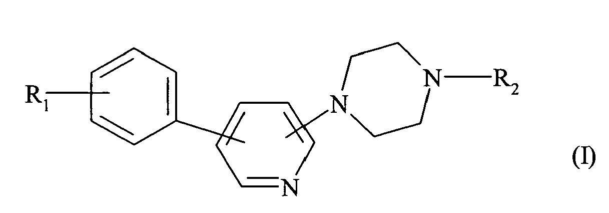 dans laquelle :
R₁ représente un groupement NR₃SO₂R₄ dans lequel :
- R₃ représente un atome d'hydrogène ou un groupement alkyle (C₁-C₆) linéaire ou ramifié,
- R₄ représente un groupement alkyle (C₁-C₆) linéaire ou ramifié, un groupement aryle ou un groupement NR₅R₆ dans lequel :
R₅ et R₆, identiques ou différents, représentent chacun un atome d'hydrogène ou un groupement alkyle (C₁-C₆) linéaire ou ramifié, cycloalkyle (C₃-C₈) ou cycloalkyl (C₃-C₈) alkyle (C₁-C₆) linéaire ou ramifié,
ou R₅ et R₆ forment ensemble avec l'atome d'azote qui les porte un cycle comportant de 5 à 8 chaînons dont un des atomes de carbone peut être remplacé par un atome d'azote, d'oxygène ou de soufre, ou par un groupement SO ou SO₂ le cycle ainsi défini pouvant être ponté par une chaîne alkyle(C₁-C₆) linéaire ou ramifiée et/ou pouvant être éventuellement substitué par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, carboxy, hydroxy, cyano, nitro, amino (éventuellement substitué par un ou plusieurs groupements alkyle (C₁-C₆) linéaire ou ramifié),
R₂ représente un groupement alkyle (C₁-C₆) linéaire ou ramifié, un groupement cycloalkyle (C₃-C₈), ou un groupement cycloalkyle (C₃-C₈) alkyle (C₁-C₆) dans lequel la partie alkyle peut être linéaire ou ramifiée,
étant entendu que :
par groupement aryle, on comprend les groupements phényle, naphtyle et biphényle, ces groupements étant éventuellement substitués par un ou plusieurs groupements, identiques ou différents, choisis parmi halogène, alkyle (C₁-C₆) linéaire ou ramifié, alkoxy (C₁-C₆) linéaire ou ramifié, polyhalogénoalkyle (C₁-C₆) linéaire ou ramifié, carboxy, hydroxy, cyano, nitro, amino (éventuellement substitué par un ou plusieurs groupements alkyle (C₁₋C₆) linéaire ou ramifié),
leurs énantiomères et diastéréoisomères ainsi que leurs sels d'addition à un acide ou à une base pharmaceutiquement acceptable.

2. Composés de formule (I), selon la revendication 1, pour lesquels R₄ représente un groupement alkyle, leurs énantiomères et diastéréoisomères ainsi que leurs sels d'addition à un acide ou à une base pharmaceutiquement acceptable.

3. Composés de formule (I), selon la revendication 1, pour lesquels R₃ représente un atome d'hydrogène, leurs énantiomères et diastéréoisomères ainsi que leurs sels d'addition à un acide ou à une base pharmaceutiquement acceptable.

4. Composés de formule (I), selon la revendication 1, pour lesquels R₅ et R₆ forment ensemble avec l'atome d'azote qui les porte un cycle comportant de 5 à 8 chaînons dont un des atomes de carbone peut être remplacé par un atome d'azote, d'oxygène ou de soufre ou par un groupement SO ou SO₂, leurs énantiomères et diastéréoisomères ainsi que leurs sels d'addition à un acide ou à une base pharmaceutiquement acceptable.

5. Composés de formule (I), selon la revendication 1, pour lesquels R₂ représente le groupement isopropyle, leurs énantiomères et diastéréoisomères ainsi que leurs sels d'addition à un acide ou à une base pharmaceutiquement acceptable.

6. Composés de formule (I), selon la revendication 1, pour lesquels R₂ représente le groupement cyclopropyle ou cyclopentyle, leurs énantiomères et diastéréoisomères ainsi que leurs sels d'addition à un acide ou à une base pharmaceutiquement acceptable.

7. Composé de formule (I), selon la revendication 1, qui est le *N*-[4-[6-(4-cyclopentyl-1-pipérazinyl)-3-pyridinyl]phényl]méthane sulfonamide, dichlorhydrate ainsi que ses sels d'addition à un acide ou à une base pharmaceutiquement acceptable.

8. Procédé de préparation des composés de formule (I), selon la revendication 1, **caractérisé en ce que** l'on utilise comme produit de départ le composé de formule (II) : 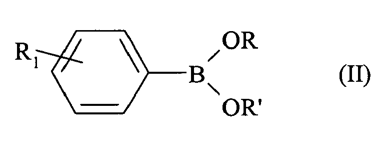 dans laquelle R₁ est tel que défini dans la formule (I) et R et R', identiques ou différents, représentent chacun un atome d'hydrogène ou un groupement alkyle (C₁-C₆) linéaire ou ramifié, ou forment ensemble une chaîne alkylène (C₁-C₆) linéaire ou ramifiée,
sur lequel on condense en présence de palladium (0) un composé de formule (III) : 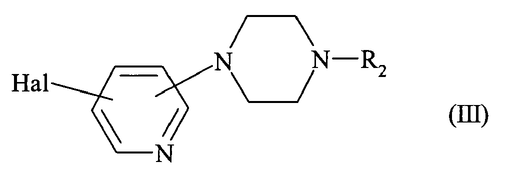 dans laquelle R₂ est tel que défini dans la formule (I) et Hal représente un atome d'halogène, pour conduire au composé de formule (I),
composé de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

9. Compositions pharmaceutiques contenant comme principe actif un composé selon l'une quelconque des revendications 1 à 7 en combinaison avec un ou plusieurs excipients ou véhicules inertes, non toxiques, pharmaceutiquement acceptables.

10. Compositions pharmaceutiques selon la revendication 9 contenant comme principe actif un composé selon l'une quelconque des revendications 1 à 7 utiles pour la synthèse d'un médicament en tant qu'antagoniste des récepteurs histaminergiques centraux de type H₃.

11. Compositions pharmaceutiques selon la revendication 9 contenant au moins un principe actif selon l'une quelconque des revendications 1 à 7 utiles, en tant que médicament, dans le traitement des déficits cognitifs associés au vieillissement cérébral et aux maladies neurodégénératives, ainsi que dans le traitement des troubles de l'humeur, des crises convulsives, du syndrome d'hyperactivité avec déficits attentionnels, de l'obésité, de la douleur et des états narcoleptiques.

12. Compositions pharmaceutiques selon la revendication 9 contenant au moins un principe actif selon l'une quelconque des revendications 1 à 7 utiles, en tant que médicament, dans le traitement des déficits cognitifs associés à la maladie d'Alzheimer, la maladie de Parkinson, la maladie de Pick, la maladie de Korsakoff, et les démences frontales et sous-corticales d'origines vasculaires ou autres.
